## Europäisches Patentamt
## European Patent Office
## Office européen des brevets

(19)

(11) Veröffentlichungsnummer: **0 032 665**
**B1**

(12)

# EUROPÄISCHE PATENTSCHRIFT

(45) Veröffentlichungstag der Patentschrift:
**12.01.83**

(51) Int. Cl.³: **C 07 C 31/20,** C 07 C 29/80

(21) Anmeldenummer: **81100042.1**

(22) Anmeldetag: **07.01.81**

(54) **Verfahren zur Herstellung von hochreinem Monoethylenglykol.**

(30) Priorität: **18.01.80 DE 3001727**

(43) Veröffentlichungstag der Anmeldung:
**29.07.81 Patentblatt 81/30**

(45) Bekanntmachung des Hinweises auf die Patenterteilung:
**12.01.83 Patentblatt 83/2**

(84) Benannte Vertragsstaaten:
**BE DE FR GB IT NL**

(56) Entgegenhaltungen:
**DE-A-2 364 151**
**DE-A-2 751 383**
**US-A-3 878 055**

(73) Patentinhaber: **HOECHST AKTIENGESELLSCHAFT,**
**Postfach 80 03 20, D-6230 Frankfurt/Main 80 (DE)**

(72) Erfinder: **Rebsdat, Siegfried, Dr., Trenbeckstrasse 24,**
**D-8261 Burg Post Neuötting (DE)**
Erfinder: **Mayer, Sigmund, Hochfellnstrasse 20,**
**D-8261 Burgkirchen/Alz (DE)**
Erfinder: **Alfranseder, Josef, Hauptstrasse 31,**
**D-8261 Hofschallern Post Markt1 (DE)**
Erfinder: **Schaffelhofer, Iwo, Dr., Ketteler Strasse 2,**
**D-8263 Burghausen/Salzach (DE)**

Verfahren zur Herstellung von hochreinem Monoethylenglykol

Die Erfindung betrifft ein Verfahren zur Herstellung von hochreinem Monoethylenglykol, bei dem Ethylenoxid und Wasser in einem Reaktor umgesetzt werden und das Reaktionsprodukt einer Destillationszone aus mehreren, jeweils durch Rohrleitungen verbundenen Destillationskolonnen zugeleitet wird, wobei mindestens die ersten beiden Kolonnen zum Abdestillieren von Wasser und leichter als Monoethylenglykol siedenden Anteilen benutzt und in den weiteren Kolonnen nacheinander Monoethylenglykol und gegebenenfalls höhere Ethylenglykole über Kopf abgezogen werden.

Es ist bekannt, Ethylenoxid, das vorzugsweise durch direkte Oxidation von Ethylen mit Sauerstoff und/oder Luft unter Verwendung von Silber-Katalysatoren erhalten worden ist, mit (einem etwa 5- bis 50fachen molaren Überschuss an) Wasser in einem Reaktor bei einer Temperatur von etwa 160 bis 230°C und einem Druck von etwa 2 bis 6 MPa zu im wesentlichen Monoethylenglykol umzusetzen, wobei diese Umsetzung (Hydratation) vorzugsweise katalysatorfrei durchgeführt und aus dem Reaktionsprodukt (Reaktionsgemisch) Monoethylenglykol destillativ gewonnen wird.

Zur destillativen (fraktionierten) Aufarbeitung wird das Reaktionsprodukt durch eine Destillationszone geleitet, die aus mehreren, mit Rohrleitungen verbundenen Kolonnen, in der Regel aus 3 bis 10, vorzugsweise aus 3 bis 7 Kolonnen besteht (vergleiche Ullmanns Enzyklopädie der technischen Chemie, 4. Auflage, Band 8, Seiten 200 bis 203. Vergleiche beispielsweise auch die deutsche Offenlegungsschrift 23 64 151).

Bei dieser bekannten destillativen Aufarbeitung des Reaktionsproduktes aus der Umsetzung von Ethylenoxid und Wasser werden zunächst in der sogenannten Entwässerungsstufe, die in der Regel aus mindestens 2 Kolonnen, vorzugsweise aus 2 bis 5 Kolonnen, besteht, das überschüssige Wasser und gegebenenfalls Verunreinigungen, deren Siedepunkte unter dem des Monoethylenglykols liegen (leichtsiedende Anteile), beispielsweise Formaldehyd, Acetaldehyd und dergleichen, abdestilliert. Die Abtrennung von Wasser und Leichtsiedern wird also stufenweise vorgenommen. Dabei wird in der Regel die erste Kolonne (erste Entwässerungsstufe) bei einem Druck von etwa 0,8 bis 2 MPa und einer Temperatur von etwa 140 bis 180°C und die weiteren Kolonnen bei einem relativ kleinen Druck oder drucklos und bei einer etwa gleichen oder etwas höheren Temperatur (im Vergleich zur ersten Kolonne) betrieben.

Nachdem aus dem Reaktionsprodukt aus Roh-Ethylenoxid und Wasser in der Entwässerungsstufe die Leichtsieder und das Wasser abdestilliert worden sind (der Gehalt an Wasser beträgt nun weniger als etwa 0,1 Gew.-%), tritt der Produktstrom in die Ethylenglykol-Destillationsstufe ein, die in der Regel aus 1 bis 3 Kolonnen besteht.

In der ersten Kolonne dieser Stufe wird bei einer Temperatur von etwa 140 bis 170°C und einem Druck von etwa 2 bis 4 kPa Monoethylenglykol über Kopf abgezogen. Der Sumpf der Monoethylenglykol-Kolonne kann in den nachfolgenden Kolonnen noch weiter fraktioniert werden, wobei beispielsweise Diethylenglykol, Triethylenglykol und höhere Ethylenglykole gewonnen werden (vergleiche genannte Literaturstelle in Ullmanns Enzyklopädie der technischen Chemie).

Monoethylenglykol, das zur Herstellung von Fasern (durch Kondensation des Glykols mit beispielsweise Dimethylterephthalat) eingesetzt wird, soll bekanntlich eine besonders hohe Reinheit aufweisen.

Als Mass für diese Reinheit wird der Transmissionswert im ultravioletten Licht (UV-Licht) der Wellenlänge 220 nm gemessen, der über 65% liegen soll.

Das nach dem oben beschriebenen, bekannten Herstellungsverfahren erhaltene Monoethylenglykol erfüllt diese Forderung nicht. Andererseits stellt gerade dieses Herstellungsverfahren einen besonders vorteilhaften Weg zur Herstellung von relativ reinem Monoethylenglykol dar.

Es sind bereits mehrere Vorschläge gemacht worden, die sich auf die Herstellung von reinem Monoethylenglykol beziehen.

So wird in der deutschen Offenlegungsschrift 16 68 052 und in der deutschen Auslegeschrift 25 58 039 ein Verfahren zur Reinigung von Ethylenglykolen beschrieben, bei dem diese Glykole durch ein Ionenaustauscherharz geleitet werden.

In der DE-OS 15 93 120 wird empfohlen, das oben beschriebene Destillationsverfahren zur Aufarbeitung des Reaktionsproduktes aus Ethylenoxid und Wasser in Gegenwart eines Antioxidationsmittels, insbesondere in Gegenwart eines aromatischen Amins oder Phenols, durchzuführen. Ferner ist in der deutschen Offenlegungsschrift 27 51 383 ein Verfahren zur Herstellung von Ethylenglykolen mit hoher Reinheit und mit niedrigen UV-Absorptionswerten, ausgehend von Ethylenoxid, beschrieben, bei dem man zur Hydratation des Ethylenoxids dasjenige Wasser verwendet, das beim Waschen des rohen Ethylenoxids vor der Hydratationsstufe angefallen ist und dem Natriumborhydrid zugesetzt worden ist.

Diese bekannten Verfahren zur Herstellung von Monoethylenglykol lassen noch insofern zu wünschen übrig, als ihre Durchführung einen zusätzlichen Arbeitsschritt in der Reinigung erfordert (wie den oben erwähnten Ionenaustausch) beziehungsweise solche Substanzen zum Reaktionsprodukt aus Ethylenoxid und Wasser vor oder während seiner Destillation zugesetzt werden müssen, bei denen die Gefahr besteht, dass sie selbst (zum Beispiel Phenol) oder Folgeprodukte von ihnen (zum Beispiel Borsäureester aus Natriumborhydrid) auch im gewonnenen Monoethylenglykol vorhanden sein könnten.

Der Erfindung liegt nun die Aufgabe zugrunde, das eingangs genannte Destillationsverfahren zur Aufarbeitung des Reaktionsproduktes aus der Umsetzung von Ethylenoxid und Wasser derart zu gestalten, dass die genannten Verfahrensnachteile nicht mehr vorliegen, und dass ein Monoethylenglykol erhalten wird, das die geforderte UV-Reinheit besitzt.

Überraschenderweise wurde nun gefunden, dass man beim eingangs genannten Verfahren dann ein hochreines (das heisst UV-reines) Monoethylenglykol erhält, wenn man die Destillation in Gegenwart von Alkaliverbindungen durchführt, so dass das in die Monoethylenglykol-Kolonne eintretende Roh-Monoethylenglykol einen pH-Wert von 7 bis 10 aufweist.

Gefunden wurde demnach ein Verfahren zur Herstellung von hochreinem Monoethylenglykol, bei dem Ethylenoxid und Wasser in einem Reaktor umgesetzt werden und das Reaktionsprodukt einer Destillationszone aus mehreren, jeweils durch Rohrleitungen verbundenen Destillations-Kolonnen zugeleitet wird, wobei mindestens die ersten beiden Kolonnen zum Abdestillieren von Wasser und den leichter als Monoethylenglykol siedenden Anteilen benutzt und in den weiteren Kolonnen nacheinander Monoethylenglykol und gegebenenfalls höhere Ethylenglykole über Kopf abgezogen werden, dadurch gekennzeichnet, dass nach dem Reaktor und vor der Kolonne, aus der das Monoethylenglykol über Kopf abgezogen wird, Verbindungen von Alkalimetallen aus der Gruppe der Alkoholate, Hydroxide, Oxide und Salze von schwachen Säuren zugegeben werden, so dass der pH-Wert des Produktes bei seinem Eintritt in die Monoethylenglykol-Kolonne auf 7 bis 10 eingestellt ist.

Die Zugabe der Alkalimetall-Verbindungen erfolgt vorzugsweise nach der ersten oder nach der zweiten Destillationskolonne (Entwässerungsstufe), das heisst, die Verbindungen werden dem Produktstrom (Roh-Monoethylenglykol) vorzugsweise in der Leitung zugesetzt, die den Sumpf der ersten Kolonne in die zweite Kolonne oder den Sumpf der zweiten Kolonne in die dritte Kolonne führt.

Der pH-Wert des Produktes (Roh-Monoethylenglykol) vor seinem Eintritt in die Monoethylenglykol-Kolonne beträgt vorzugsweise 7,2 bis 8,9. Besonders bevorzugt ist ein pH-Wert von 7,5 bis 8,5. Das Messen des pH-Wertes erfolgt zweckmässigerweise mit einem üblichen pH-Meter (unter Zugabe von destilliertem Wasser). Die Probe wird vorzugsweise an einer Stelle in der Leitung entnommen, die den Produktstrom aus dem Boden der letzten Kolonne der Entwässerungsstufe in die Monoethylenglykol-Kolonne einleitet.

Zur Einstellung des pH-Wertes werden erfindungsgemäss Alkalialkoholate, Alkalihydroxide, Alkalioxide und/oder Alkalisalze von schwachen Säuren eingesetzt, wobei die entsprechenden Natrium- und Kaliumverbindungen bevorzugt sind.

Als Alkoholate werden vorzugsweise diejenigen mit 1 bis 3 C-Atomen, wie Natriummethylat und Natriummethylat, eingesetzt.

Als Alkalisalze von schwachen Säuren werden vorzugsweise Salze der Kohlensäure, beispielsweise Kaliumcarbonat, Kaliumhydrogencarbonat und Natriumcarbonat, eingesetzt.

Von den erfindungsgemäss einzusetzenden Alkalimetall-Verbindungen werden Carbonate und Hydroxide bevorzugt. Besonders bevorzugt ist Natriumhydroxid (NaOH) und Kaliumhydroxid (KOH).

Die Menge an Alkalimetallverbindung, die zur Erreichung des erfindungsgemäss angestrebten pH-Wertes erforderlich ist, kann in weiten Grenzen variieren. Sie beträgt in der Regel 0,1 bis 500 mg, vorzugsweise 1 bis 100 mg pro kg von zur Hydratation eingesetztem Ethylenoxid und Wasser.

Die Alkalimetall-Verbindung wird zweckmässigerweise in Form einer wässrigen, alkoholischen, wässrig-alkoholischen oder wässrig-glykolischen Lösung zugegeben.

Im Falle der Verwendung von Alkoholen als Lösungsmittel werden vorzugsweise Methanol, Ethanol, Propanol und/oder Isopropanol eingesetzt.

Die Konzentration der verwendeten Lösungen ist nicht kritisch. Sie kann in weiten Grenzen variieren. Im allgemeinen werden 3- bis 30gew.-%ige, vorzugsweise 15- bis 25gew.-%ige, Lösungen eingesetzt.

Die Lösungen werden zweckmässigerweise über eine übliche Dosiereinrichtung eingespeist, die in der Regel durch das pH-Meter gesteuert wird.

Mit dem erfindungsgemässen Verfahren wird ein Monoethylenglykol mit einer überraschend hohen Reinheit erhalten. Dieses Monoethylenglykol besitzt eine Transmission bei 220 nm UV-Licht, die im allgemeinen weit über dem geforderten Spezifikationswert liegt. Darüber hinaus enthalten sowohl das so hergestellte Monoethylenglykol als auch die höheren Ethylenglykole (für die ebenfalls hohe Reinheit gewünscht wird) keine artfremden Verbindungen. Ein weiterer wesentlicher Vorteil des erfindungsgemässen Verfahrens besteht darin, dass dabei auch das Problem des Anfalls von Ethylenglykol enthaltenden wässrigen Mutterlaugen, die bei der Herstellung von Ethylenoxid durch direkte Oxidation von Ethylen zwangsläufig erhalten werden (vergleiche deutsche Auslegeschrift 25 58 039), weitgehend gelöst wird. Beim erfindungsgemässen Verfahren ist es nämlich möglich, dem Roh-Monoethylenglykol eine relativ grosse Menge von diesen Mutterlaugen zuzugeben, ohne dass dadurch die UV-Reinheit des hergestellten Monoethylenglykols nennenswert vermindert würde. Beim eingangs beschriebenen bekannten Destillationsverfahren tritt in den einzelnen Destillationskolonnen oft Korrosion auf. Beim erfindungsgemässen Verfahren ist hingegen keine Korrosion der Destillationskolonnen mehr festzustellen, da sie durch die erfindungsgemässe Zugabe von Alkaliverbindungen offensichtlich ausgeschaltet ist.

Das erfindungsgemässe Verfahren ist darüber hinaus einfach, wirtschaftlich und kann im Rahmen der bekannten Destillation und somit in den dafür bereits bestehenden Anlagen durchgeführt werden.

Das nach dem erfindungsgemässen Verfahren hergestellte hochreine Monoethylenglykol wird insbesondere zur Herstellung von Fasern (Polyesterfasern) verwendet.

Die Erfindung wird nun anhand einer Zeichnung und in Beispielen noch näher erläutert.

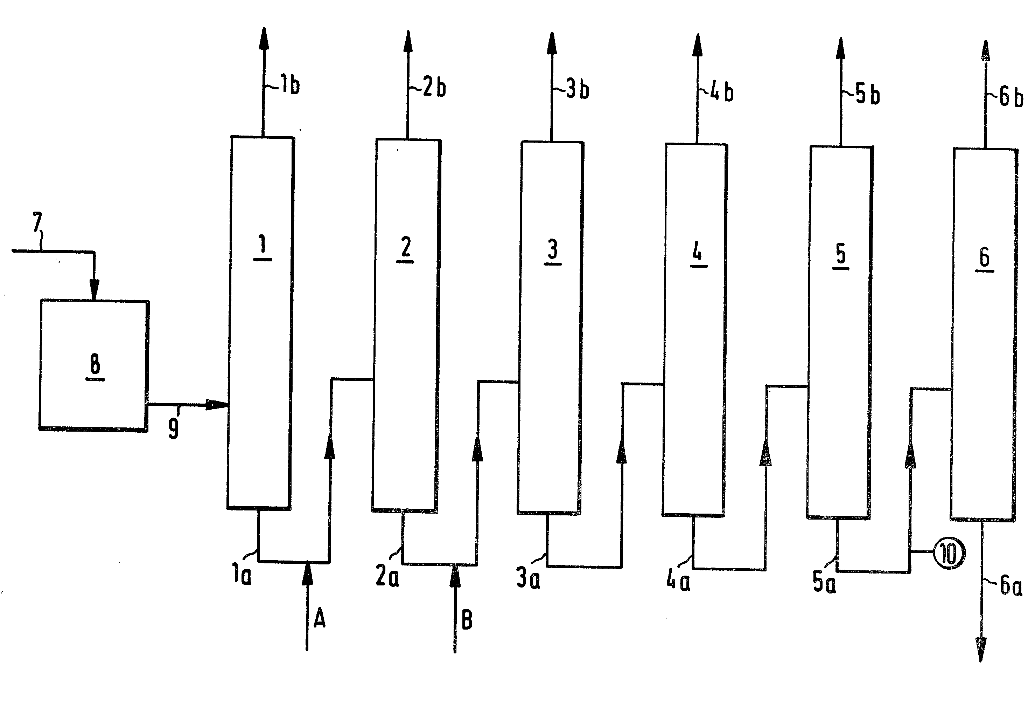

Fig. 1 zeigt ein vereinfachtes Fliesschema des eingangs beschriebenen bekannten Hydratations- und Destillations-Verfahrens.

Im Reaktor 8 werden Ethylenoxid (Roh-Ethylenoxid) und Wasser, die durch die Leitung 7 herangeführt werden, umgesetzt. Das Reaktionsprodukt wird durch die Leitung 9 zu den Kolonnen 1 bis 6 geführt, die über die Leitungen 1a bis 5a miteinander verbunden sind. Die Kolonnen 1 bis 5 stellen die Entwässerungsstufen und die Kolonne 6 die Monoethylenglykol-Kolonne dar.

Die Kolonnen 1 bis 5 dienen zum Abdestillieren der Leichtsieder und des Wassers aus dem Reaktionsprodukt. Leichtsieder und Wasser werden über die Leitungen 1b bis 5b aus der jeweiligen Kolonne 1 bis 5 abgeführt. Das Roh-Monoethylenglykol, das über die Leitung 5a in die Kolonne 6 eintritt, besitzt nur noch weniger als 0,1 Gew.-% Wasser. Aus dieser Kolonne wird schliesslich das angestrebte Monoethylenglykol destilliert. Es wird über die Leitung 6b abgeführt. Das Sumpfprodukt der Kolonne 6 besteht aus Di-, Tri- und höheren Ethylenglykolen. Es wird über die Leitung 6a entfernt.

Die Stelle, an der vorzugsweise gemäss Erfindung Alkalimetall-Verbindungen eingespeist werden – so dass das Roh-Monoethylenglykol, das zur Monoethylenglykol-Kolonne geführt wird, einen pH-Wert von 7 bis 10 aufweist – sind in Fig. 1 mit einem Pfeil, der die Buchstaben A und B trägt, gekennzeichnet. Das pH-Meter, das in einem Zweigstrom in der Leitung, die zur Monoethylenglykol-Kolonne führt, angebracht ist, ist mit einem Kreis und der Nummer 10 gekennzeichnet.

Beispiel 1 (Vergleichsbeispiel)

12 000 kg Roh-Ethylenoxid, bestehend aus 33 Gew.-% Ethylenoxid, 1 Gew.-% Aldehyd (Summe der Aldehyde, vorzugsweise Formaldehyd plus Acetaldehyd) und 66 Gew.-% Wasser, wurden mit 32 000 kg Wasser vermischt und kontinuierlich in einem üblichen Druckreaktor (Hydratationsreaktor) bei 220°C und 5 MPa umgesetzt. Das Reaktionsprodukt wurde der Destillationszone aus 6 Kolonnen gemäss Fig. 1 zugeleitet.

In der ersten Kolonne wurde bei einem Druck von 1,5 MPa und einer Temperatur von 178°C (Sumpftemperatur) ein Gemisch aus Wasser und niedrigsiedenden Verunreinigungen abdestilliert. Das Sumpfprodukt der ersten Kolonne wurde in den folgenden (vier) Kolonnen weiter entwässert: Die zweite Kolonne wurde bei 145°C und Atmosphärendruck (drucklos), die dritte Kolonne bei 160°C und Atmosphärendruck, die vierte Kolonne bei 150°C und 50 kPa und die fünfte Kolonne bei 155°C und 25 kPa betrieben. Das Sumpfprodukt der fünften Kolonne, das einen Wassergehalt von wengier als 0,1 Gew.-% hatte, wurde über die Leitung 5a in die Kolonne 6 (Monoethylenglykol-Kolonne) geleitet: Die Rektifikation erfolgte bei einer Temperatur von 158°C und einem Druck von 3 kPa.

Das so erhaltene Monoethylenglykol wurde in einem üblichen UV-Messgerät bei der Wellenlänge 220 nm auf seine Transmission getestet. Sie betrug 57%.

Der pH-Wert des Sumpfproduktes der fünften Entwässerungsstufe (Kolonne 5) – gemessen mittels eines üblichen pH-Meters 10 (vergleiche Fig. 1) unter Zusatz von Wasser – betrug 6,3.

Beispiel 2

Es wurde wie in Beispiel 1 vorgegangen, wobei jedoch in die Leitung 1a 200 g NaOH pro Stunde – das sind 4,5 mg NaOH pro kg eingesetztes Ethylenoxid und Wasser (insgesamt 44 000 kg) und pro Stunde – in Form einer 20gew.-%igen wässrigen Lösung mittels einer üblichen Dosiereinrichtung über die Leitung A zugespeist wurde (vergleiche Fig. 1). Der pH-Wert des Sumpfproduktes der fünften Kolonne betrug 8,0.

Der Wert der UV-Transmission bei 220 nm des über Kopf der sechsten Kolonne abgezogenen Monoethylenglykols war 87%.

Beispiel 3

Es wurde wie in Beispiel 2 vorgegangen, wobei jedoch 100 g NaOH pro Stunde – das sind 2,3 mg NaOH pro kg eingesetztes Ethylenoxid und Wasser, und pro Stunde – zugespeist wurde.
pH-Wert des Sumpfproduktes der fünften Kolonne: 7,6,
UV-Transmission: 79%.

Beispiel 4

Es wurde wie in Beispiel 2 vorgegangen, wobei jedoch 300 g NaOH pro Stunde – das sind 7,0 mg NaOH pro kg eingesetztes Ethylenoxid und Wasser, und pro Stunde – zugespeist wurde.
pH-Wert des Sumpfproduktes der fünften Kolonne: 8,9,
UV-Transmission: 82%.

Beispiel 5

Es wurde wie in Beispiel 2 vorgegangen, wobei jedoch 50 g NaOH pro Stunde – das sind 1,2 mg NaOH pro kg eingesetztes Ethylenoxid und Wasser, und pro Stunde – zugespeist wurde.
pH-Wert des Sumpfproduktes der fünften Kolonne: 7,2,
UV-Transmission: 67%.

Beispiel 6

Es wurde wie in Beispiel 1 vorgegangen, wobei jedoch in die Leitung 2a 2000 g NaOH pro Stunde – das sind 45,5 mg NaOH pro kg eingesetztes Ethylenoxid und Wasser, und pro Stunde – in Form einer 20gew.-%igen wässrigen Lösung mittels einer üblichen Dosiereinrichtung über die Leitung B zugespeist wurde (vergleiche Fig. 1).

Der pH-Wert des Sumpfproduktes der fünften Kolonne betrug 9,5.

Der Wert der UV-Transmission bei 220 nm des über Kopf der sechsten Kolonne abgezogenen Monoethylenglykols war 73%.

Beispiel 7

Es wurde wie im Beispiel 6 vorgegangen, wobei jedoch 200 g NaOH pro Stunde – das sind 4,5 mg NaOH pro kg eingesetztes Ethylenoxid und Wasser, und pro Stunde – zugespeist wurde.

pH-Wert des Sumpfproduktes der fünften Kolonne: 7,8,

UV-Transmission: 78%.

## Patentansprüche

1. Verfahren zur Herstellung von hochreinem Monoethylenglykol, bei dem Ethylenoxid und Wasser in einem Reaktor umgesetzt werden und das Reaktionsprodukt einer Destillationszone aus mehreren, jeweils durch Rohrleitungen verbundenen Destillationskolonnen zugeleitet wird, wobei mindestens die ersten beiden Kolonnen zum Abdestillieren von Wasser und den leichter als Monoethylenglykol siedenden Anteilen benutzt werden und in den weiteren Kolonnen nacheinander Monoethylenglykol und gegebenenfalls höhere Ethylenglykole über Kopf abgezogen werden, dadurch gekennzeichnet, dass nach dem Reaktor und vor der Kolonne, aus der das Monoethylenglykol über Kopf abgezogen wird, Verbindungen von Alkalimetallen aus der Gruppe der Alkoholate, Hydroxide, Oxide und Salze von schwachen Säuren zugegeben werden, so dass der pH-Wert des Produktes bei seinem Eintritt in die Monoethylenglykol-Kolonne auf 7 bis 10 eingestellt ist.

2. Verfahren nach Anspruch 1, dadurch gekennzeichnet, dass die Alkalimetall-Verbindungen nach der ersten oder zweiten Destillationskolonne zugegeben werden.

3. Verfahren nach Anspruch 1, dadurch gekennzeichnet, dass ein pH-Wert von 7,2 bis 8,9 eingestellt wird.

4. Verfahren nach Anspruch 1, dadurch gekennzeichnet, dass ein pH-Wert von 7,5 bis 8,5 eingestellt wird.

5. Verfahren nach Anspruch 1, dadurch gekennzeichnet, dass die Verbindungen von Alkalimetallen in Form einer 3- bis 30gew.-%igen wässrigen Lösung zugegeben werden.

6. Verfahren nach Anspruch 5, dadurch gekennzeichnet, dass Alkalimetallhydroxide und/oder Alkalimetallcarbonate eingesetzt werden.

7. Verfahren nach Anspruch 5, dadurch gekennzeichnet, dass Natrium- und/oder Kaliumhydroxid eingesetzt wird.

## Claims

1. A process for the manufacture of extremely pure monoethylene glycol by reacting ehtylene oxide and water in a reactor and transferring the reaction product to a distillation zone comprising a plurality of distillation columns interconnected by tubes, at least the first two columns being used for distilling off water and the components having a boiling point below that of monoethylene glycol and in the following columns monoethylene glycol and higher ethylene glycols, if any, being distilled at the head, which comprises adding to the reaction products behind the reactor and before the column from which the monoethylene glycol is distilled at the head, compounds of alkali metals selected from the group consisting of the alcoholates, hydroxides, oxides and salts of weak acids, in an amount such that on entering the monoethylene glycol column the product has a pH of 7 to 10.

2. The process as claimed in claim 1, wherein the alkali metal compounds are added behind the first or second distillation column.

3. The process as claimed in claim 1, wherein the pH is adjusted to a value of 7.2 to 8.9.

4. The process as claimed in claim 1, wherein the pH is adjusted to a value of 7.5 to 8.5.

5. The proces as claimed in claim 1, wherein the compounds of alkali metals are added in the form of a 3 to 30% by weight aqueous solution.

6. The process as claimed in claim 5, wherein the alkali metal compound is a hydroxide and/or a carbonate.

7. The process as claimed in claim 5, wherein sodium and/or potassium hydroxide is used.

## Revendications

1. Procédé de préparation de monoéthylène-glycol de haute pureté, selon lequel on fait réagir l'oxyde d'éthylène et l'eau dans un réacteur et l'on achemine le produit de la réaction vers une zone de distillation formée de plusieurs colonnes de distillation reliées à chaque fois par des tuyaux, en utilisant au moins les deux premières colonnes pour chasser par distillation l'eau et les fractions à plus bas point d'ébullition que le monoéthylène-glycol et en retirant en-tête des colonnes suivantes successivement le monoéthylène-glycol et éventuellement des éthylène-glycols supérieurs, procédé caractérisé en ce que l'on ajoute, en aval du réacteur et en amont de la colonne dont on retire en-tête le monoéthylene-glycol, des composés de métaux alcalins choisis dans l'ensemble formé par les alcoolates, les hydroxydes, les oxydes et les sels d'acides faibles, de sorte que le pH du produit soit ajusté, à son entrée dans la colonne de distillation du mono-éthylène-glycol, à une valeur comprise entre 7 et 10.

2. Procédé selon la revendication 1, caractérisé en ce qu'on ajoute les composés de métaux alcalins en aval de la première ou de la seconde colonne de distillation.

3. Procédé selon la revendication 1, caractérisé en ce qu'on établit un pH de 7,2 à 8,9.

4. procédé selon la revendication 1, caractérisé en ce qu'on établit un pH de 7,5 à 8,5.

5. Procédé selon la revendication 1, caractérisé en ce qu'on ajoute les composés de métaux alcalins sous forme d'une solution aqueuse à 3 à 30% en poids.

6. Procédé selon la revendication 5, caractérisé en ce qu'on utilise des hydroxydes de métaux alcalins et/ou des carbonates de métaux alcalins.

7. Procédé selon la revendication 5, caractérisé en ce qu'on utilise l'hydroxyde de sodium et/ou de potassium.

0 032 665